# EUROPEAN PATENT APPLICATION

(11) **EP 1 208 909 A2**
(43) Date of publication of application: **29.05.2002**
(21) Application number: 01127937.9
(22) Date of filing: 23.11.2001
(51) Int. Cl.: B01J 19/00, G01N 33/543, C12Q 1/68

(54) **Biomolecule microarray support, biomolecule microarray using the support, and method of fabricating the support**

(30) Priority: 24.11.2000 JP 2000358121
(71) Applicant: RIKEN, Wako-shi, Saitama 351-0198 (JP); WASEDA UNIVERSITY, Shinjuku-ku, Tokyo 169-8050 (JP)
(72) Inventor: Tashiro, Hideo, Wako-shi, Saitama, 351-0198 (JP); Kondoh, Yasumitsu, Wako-shi, Saitama, 351-0198 (JP); Kitsunai, Tokuji, Wako-shi, Saitama, 351-0198 (JP); Takenaka, Shigeori, Koga-shi, Fukuoka, 811-3114 (JP); Matsumoto, Kazuko, c/o School of Science and Eng., Tokyo, 169-8555 (JP); Nojima, Takahiko, c/o Advanced Research Institute, Tokyo, 169-8555 (JP)
(74) Representative: Grosse, Wolfgang, Dipl.-Ing.

(57) **Abstract**

A biomolecule microarray support (11) for spotting solutions containing probe biomolecules on the surface and immobilizing the probe biomolecules in the solutions to the surface, characterized in that a plurality of small-sized probe biomolecule-attachable spots (101) are arrayed in a regular arrangement on the surface of the support (11). A biomolecule microarray, made by spotting solutions containing probe biomolecules on the biomolecule-attachable spots (101) on the support (11) and immobilizing the biomolecules to the spots (101), can be used for quantitative analysis because of uniform and unvarying numbers of probe biomolecules bound to the spots (101). It also makes possible high S/N ratio measurement because of prevented nonspecific adsorption of target biomolecules to the surface of the support (11) other than the detection spots (101). The biomolecule microarray support (11) is fabricated precisely and efficiently by the method of the present invention using the photolithography and etching technique.

## Description

### Field of the invention

The present invention belongs to the field of biomolecule detection technique that detects a target biomolecule by using as the probe a single-strand biomolecule with the base sequence complementary to that of the target biomolecule to be detected, letting sample biomolecules to hybridize to the probe, and detecting the presence of double strands formed by the hybridization of the target biomolecule to the probe. More specifically, the present invention relates to a biomolecule microarray support, biomolecule microarray using the support, and method of fabricating the support.

### BACKGROUND

DNA microarrays (also referred to as DNA chips) are used as a powerful tool for detecting biomolecules such as DNA or RNA in samples derived from living bodies. By using DNA microarrays, it is possible to carry out hundreds to tens of thousands of times of detection or sequencing processes together in a single process.

DNA microarrays have hundreds to tens of thousands of detection points (spots) arrayed in a regular arrangement on a several square centimeters to ten and several square centimeters support such as a glass slide or silicon chip. At each detection point, a single-strand nucleic acid polymer (gene fragment) with one known base sequence is attached to the support. In other words, a large number of nucleic acid probes with different base sequences are arrayed on a DNA microarray. An aqueous solution of a nucleic acid sample labeled with a fluorophore is applied to such a DNA microarray, and then only the nucleic acid polymers in the sample which have base sequences complementary to those of the probes hybridize to the corresponding probes. The DNA microarray is then washed, and only the target nucleic acid polymers hybridized to the probes remain on the DNA microarray. Next, the spots on the microarray are illuminated with excitation light to detect fluorescent light emitted by the fluorophore in the target nucleic acid polymers hybridized to the probes on individual spots. By thus examining whether fluorescent light is emitted from each spot on the microarray, it can be decided whether target nucleic acid polymers are present in the nucleic acid sample.

DNA microarrays can be roughly divided into the two types according to the fabricating methods: photolithographed-type and spotted-type.

Photolithographed-type DNA microarrays are made by synthesizing a large number of DNA (oligonucleotides) with different base sequences on a support by the photolithography technology used in the fabrication of semiconductor integrated circuits. DNA microarrays with high-density DNA detection points are already put to practical use (U.S.P. 5,744,305 and U.S.P. 5,445,934).

On the other hand, spotted-type microarrays are fabricated by spotting drops of solutions containing probe DNA prepared beforehand on a support which is usually a slide glass (or membrane) with the surface coated with an immobilizing agent (polylysine or aminosilane) in whole, and then drying up (U.S.P. 5,807,522).

The two types of DNA microarrays described above have the following different features.

Photolithographed-type DNA microarrays have the advantage of a high measurement sensitivity and assured reproducibility because DNA detection points can be made very small and probe DNA can be grown uniformly. It is therefore usable for the SNP (Single Nucleotide Polymorphism) analysis. However, one mask is required for adding each of four bases A, T, G, and C to the molecules being synthesized, and hence 80 masks are required for synthesizing 20-base long probes, for example. Since masks are so expensive, costing several thousands dollars per mask, and a total cost of as much as tens of thousands of dollars are needed for fabricating a DNA microarray. Photolithographed-type DNA microarrays are therefore used only by some research laboratories.

Since spotted-type microarrays are fabricated by spotting droplets containing probe DNA on a support and drying up, the density and uniformity of the DNA probes attached to the support are not assured. In other words, the DNA detection spots are not uniform in size and shape, causing a variation in the amounts of DNA attached to the spots. For this reason, spotted-type microarrays can be used only for qualitative analysis, and is not suitable for quantitative analysis. That is, by spotted-type microarrays, it is possible to detect the presence of detection spots at which a target biomolecule is hybridized to the probe, but not possible to measure the amount of the target biomolecule hybridized at each spot. Further, target biomolecules nonspecifically attach to the surface of the microarray around the detection spots because of the presence of the immobilizing agent and cause a decrease in the S/N ratio of measurement by increased noise.

The object of the present invention is therefore to provide a spotted-type biomolecule microarray that can be used for quantitative analysis and makes possible the detection of hybridization at a high S/N ratio.

### SUMMARY OF THE INVENTION

The above-mentioned object of the present invention is achieved by the following biomolecule microarray support and the biomolecule microarray fabricated using the biomolecule microarray support.

The biomolecule microarray support of the present invention is characterized in that a plurality of small-sized probe biomolecule-attachable spots are arrayed in a regular arrangement on the surface of the support.

The probe biomolecule-attachable spots have a layer of any one of biomolecule-immobilizing agents including avidin, streptavidin, biotin, amino group, carbonyl group, hydroxyl group, succinimide group, maleimide group, and thiol group.

The support may be a glass plate, silicon chip, plastic plate, gold or gold-coated plate, or silver or silver-coated plate.

The probe biomolecule-attachable spots may have avidin molecules bound to the ends of the biotin molecules bound to the surface of the support in a single layer.

The spot size is preferably within the range of 50 to 200 µm. The space between the neighboring spots is preferably within the range of 100 to 500 µm. Here, the spot size means the diameter if the shape of the spots is a circle and the length of each side if the shape of the spots is a square.

It is preferable that the shape of the probe biomolecule-attachable spots is about the same as that of the pixel elements of a semiconductor imaging device used for image acquisition of the detection spots of biomolecule microarrays.

The biomolecule microarray of the present invention is characterized in that probe biomolecules are bound to the probe biomolecule-attachable spots of the supports described above.

The probe biomolecules used for the biomolecule microarray of the present invention are DNA, RNA, PNA, or protein. The probe biomolecules are labeled with biotin and immobilized to the probe biomolecule-attachable spots by biotin-avidin binding.

The biomolecule microarray support fabricating method of the present invention is characterized by comprising steps by which the probe biomolecule-attachable spots are formed only on the specific areas of the surface by the photolithography and etching technique.

These and other objects, features, and advantages of the present invention will become more apparent and be more clearly understood from a review of the following detailed description of the disclosed embodiments and by reference to the appended drawings and claims.

### BRIEF DESCRIPTION OF THE DRAWINGS

Fig. 1 is a schematic representation of an embodiment of the biomolecule microarray support fabricating method of the present invention.
Fig. 2 is schematic diagram showing the process in which avidin molecules bind to the ends of biotin molecules on each probe biomolecule-attachable spot.
Fig. 3 is a schematic diagram showing a fabricating method of the DNA microarray of the present invention.
Fig. 4 is schematic diagram showing the process in which probe DNA molecules (biotinylated DNA) bind to the avidin molecules bound to each probe biomolecule-attachable spot.
Fig. 5 is a graph showing the relationship between the amount (concentration) of DNA in the solution spotted on one probe biomolecule-attachable spot and the amount of DNA immobilized to the spot.

### DETAILED DESCRIPTION OF PREFERRED EMBODIMENTS

Referring now to the drawings, preferred embodiments of the present invention are described below in detail.

First, the biomolecule microarray support of the present invention (hereinafter simply referred to as surface-modified support) is described.

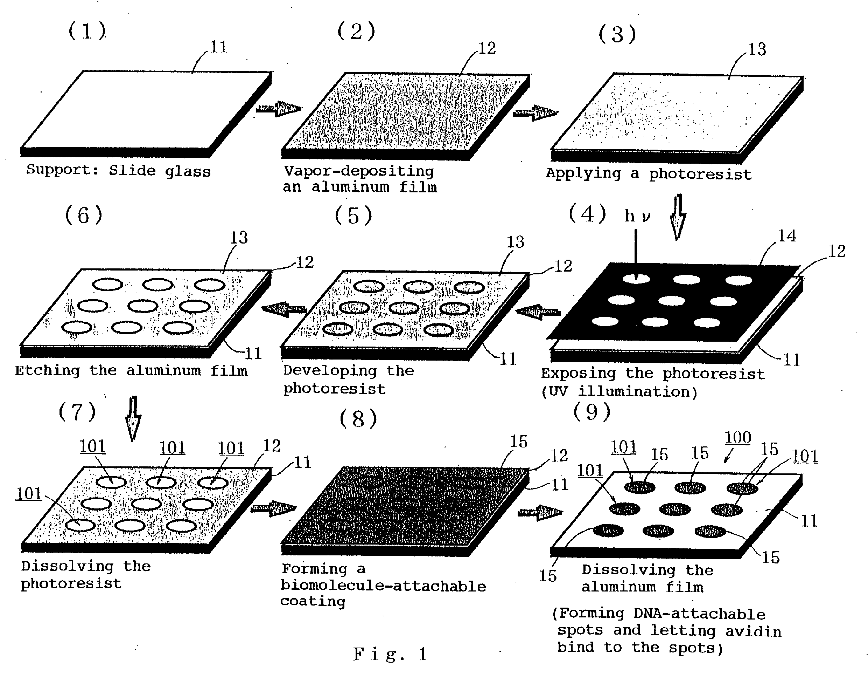

Fig. 1 is a schematic representation showing an embodiment of the surface-modified support fabricating method of the present invention. In Fig. 1, a slide glass 11 is surface-modified by the fabricating process as shown to become a surface-modified support 100 which has a modified surface such that probe DNA molecules attach only to specific areas (probe DNA-attachable spots) 101 and does not attach to the surface other than the specific areas.

This surface-modified support fabricating process comprises the following steps:
(1) Support cleaning step: Cleans a slide glass support 11 to get rid of contamination.
(2) Aluminum film vapor-depositing step: Forms an aluminum film 12 over the surface of the support 11.
(3) Photoresist applying step: Coats the surface of the aluminum film 12 with a positive photoresist.
(4) Exposing step: Exposes the photoresist only on the specific areas 101 of the surface of the support from step (3) to UV light through a photomask 14.
(5) Developing step: Develops the photoresist 13 on the support from step (4). The photoresist 13 on the specific areas 101 is removed by this step.
(6) Etching step: Etches the aluminum film on the support from step (5). The aluminum film 12 on the specific areas 101 is removed by this step.
(7) Resist removing step: Removes the photoresist 13 on the support from step (6) by dissolving it in acetone. Consequently, the surface of the slide glass support 11 becomes exposed only on the specific areas 101 by this step.
(8) DNA-immobilizing agent coating step: Applies to the surface of the support from step (7) an agent for forming a DNA-immobilizing layer 15 which immobilizes probe DNA on the surfaces of the specific areas 101. Specifically, this step comprises two steps: the first step introducing amino groups onto the surface of the support by amino-silanization, and the second step introducing biotin to the amino groups on the surface of the support by biotin-succinimide.
(9) DNA-attachable spot forming step: Removes the aluminum film 12 on the support from step (8) by dissolving it with an acid, alkali, or chelate agent. By this step, the DNA-immobilizing layer 15 formed in step (8) remains only on the specific areas of the surface of the slide glass support 11 as the DNA-attachable spots.
(10) Avidin binding step: Applies an avidin solution to the surface of the support from step (9) to let avidin molecules bind to the ends of the biotin molecules on the DNA-immobilizing layer 15 on the specific areas 101 (DNA-attachable spots).

By the above steps (1) to (10), a DNA microarray 100 which has avidin immobilized in a single layer only to the specific areas (DNA-attachable spots) 101 on the surface of the slide glass 11. The diameter of each spot is preferably equal to or smaller than 200 µm, and the space between the neighboring spots is preferably equal to or smaller than 400 µm.

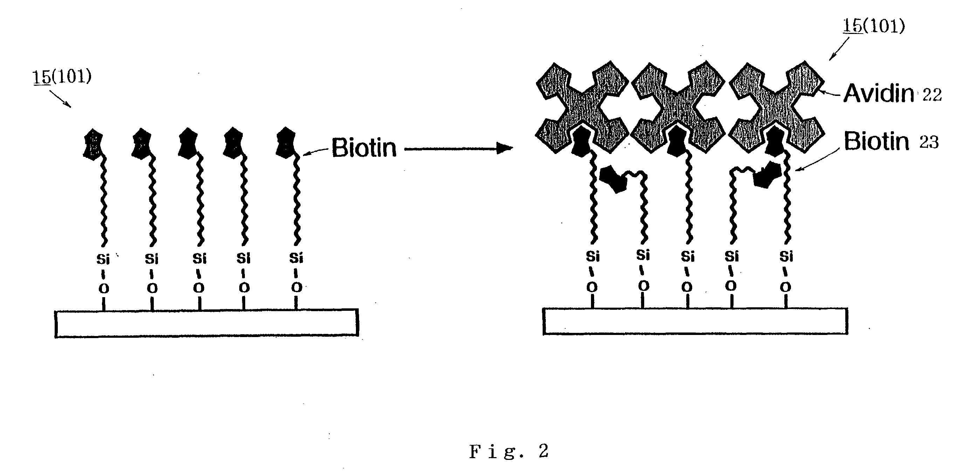

Fig. 2 shows the process in which avidin molecules bind to the ends of the biotin molecules on the DNA-immobilizing layer 15 in a single layer.

Since the DNA-attachable spots (specific areas) 101 formed on the surface of a glass support are uniform in size and shape, the numbers of the biotin molecules 23 bound to the DNA-attachable spots 101 are approximately equal. Therefore, the numbers of the avidin molecules 22 bound to individual DNA-attachable spots 101 become equal. Specifically, if there is a small variation in the numbers of the biotin molecules bound to the DNA-attachable spots 101, the numbers of the avidin molecules which bind to the DNA-attachable spots 101 becomes equal because avidin molecule is much larger than biotin molecule.

The shape and size of the DNA-attachable spots 101 and the space between the neighboring spots can be changed as desired by altering the photomask used in the exposing step.

Consequently, the number of the avidin molecules which bind to the DNA-attachable spots 101 can be adjusted as desired by altering the photomask.

### [Example]

### (1) Support cleaning step

A slide glass plate (S111, Matsunami) was soaked in acetone and washed by ultrasonic washing for 30 minutes. The glass plate was then washed with deionized water three times. Next, the slide glass plate was soaked in hydrogen peroxide/concentrated sulfuric acid (1:1) and washed by ultrasonic washing for 30 minutes. The slide glass plate was then washed with deionized water three times. Next, the glass plate was washed by ultrasonic washing with very high-purity water (Milli-Q WATER) for 30 minutes. The slide glass plate was then washed with very high-purity water (Milli-Q WATER) three times, and dried up in an oven at 60 °C for 20 minutes.

### (2) Aluminum film vapor-depositing step

An aluminum film was formed over the surface of one side of the slide glass plate by means of a vapor deposition system (ULVAC). The thickness of the aluminum film was approximately 300 nm.

### (3) Photoresist applying step

A few drops of positive photoresist (S1813, Shipley) were deposited on the aluminum-coated slide glass plate and spread over the surface in a thickness of 1 µm by means of a spin coater (Mikasa). The slide glass plate with photoresist was baked at 120 °C for 20 minutes.

### (4) Exposing step

UV light was shown onto the glass plate with photoresist through a photomask with DNA-attachable spot pattern, and only the photoresist under the spots was exposed to the UV light.
An exposing apparatus made on an experimental basis which can perform scanning exposure with third-harmonic-generated light (wavelength: 355 nm, laser power: 80 mW) of YAG laser was used. The scanning time was 120 seconds (30mm x 80mm area), which was the optimum scanning time determined by experiments.

### (5) Developing step

After the exposure, the slide glass plate was soaked in a developing solution (CD-26, Shipley) for 35 seconds, and then washed with very high-purity water (Milli-Q WATER). As the result, only the photoresist under the spots exposed to UV light dissolved and the aluminum film was exposed in those areas.

### (6) Etching step

The slide glass plate with the photoresist having the pattern transferred from the photomask was soaked in an etching solution (phosphoric acid/acetic acid/nitric acid/Milli-Q water (16:2:1:1)) for 3 minutes to dissolve the aluminum film in the spot areas. The glass plate was then washed.

### (7) Resist removing step

The photoresist on the slide glass plate was dissolved by the first, second, and third washing with acetone.

### (8) DNA-immobilizing agent coating step

The slide glass plate with the surface exposed in the spot areas and covered by the aluminum film in the other area was soaked in 1% aminosilane solution (3-(2-aminoethylaminopropyl)-trimethoxysilane in 95% acetone) for 10 minutes. The glass plate was then washed for 10 minutes three times to remove the aminosilane not bound to the glass surface, and dried up in an oven at 110 °C for 40 minutes. After the drying, the glass plate was left to cool to room temperature.

Next, the slide glass plate was soaked in 5mg/ml biotin-long arm-NHS solution (in 0.05M NaHCO₃, pH8.6) at room temperature for 4 hours. The glass plate was then washed with Milli-Q water and vacuum-dried.

### (9) DNA-attachable spot forming step

The slide glass plate was soaked in an etching solution with ultrasonic agitation for 5 minutes, and then soaked in very-high purity water (Milli-Q water) with ultrasonic agitation. By this process, the aluminum was dissolved, and the biotin thereon mechanically peeled off. As the result, a biotinylated layer was formed only on the DNA-attachable spots.

### (10) Avidin binding step

Next, 0.1mg/ml avidin solution (Cy3-labeled streptavidin, Buffer 1xSSPE, pH7.3, Vector) was placed over the slide glass plate with the biotinylated DNA-attachable spots formed, and left at room temperature for 30 minutes. The glass plate was then washed with buffer 1xSSPE (pH7.3) for 10 minutes twice. Finally, the glass plate was washed with very-high purity water (Milli-Q water) five times and vacuum-dried. Thus a biomolecule microarray with avidin bound to the DNA-attachable spots was obtained.

Next, the DNA microarray of the present invention is described below.

The DNA microarray of the present invention is fabricated by spotting solutions containing probe DNAs with different base sequences on the DNA-attachable spots 101 of the surface-modified support 100 fabricated by the method shown in Fig. 1.

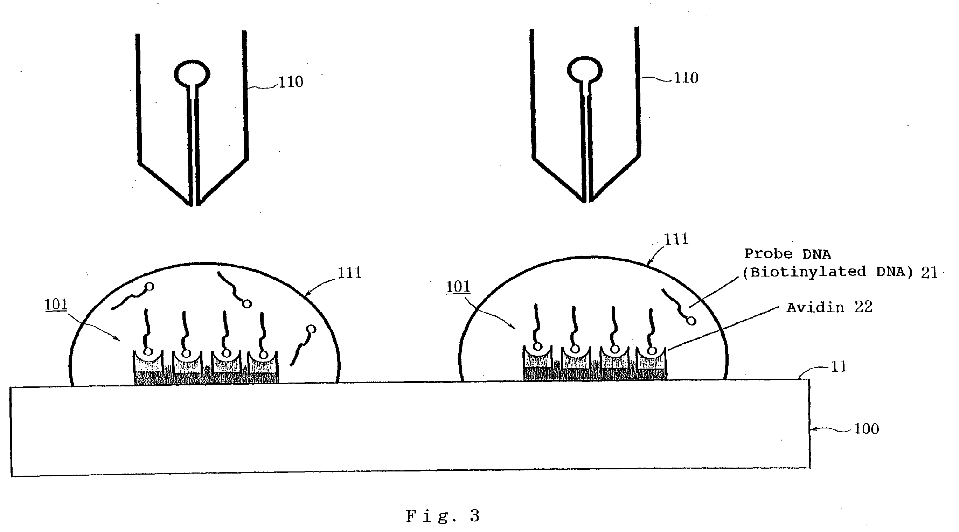

Fig. 3 shows a method of fabricating the DNA microarray of the present invention. In Fig. 3, a solution 111 containing probe DNA molecules 21 is spotted by an arrayer 110 on the support 100.

The probe DNA molecules 21 are labeled with biotin (biotinylated DNA) beforehand. The arrayer 110 has a structure which can hold a fixed amount of a solution 111 by capillary action, and a uniform amount of the solution 111 is dispensed on each DNA-attachable spot 101 by pressing the tip of the arrayer 110 holding the solution 111 onto the DNA-attachable spot 101.

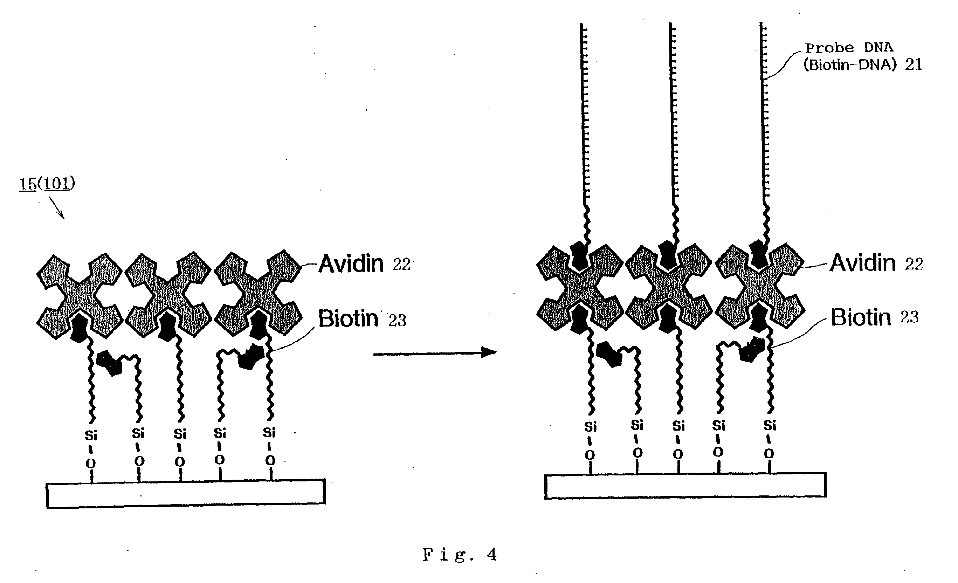

As the result, the probe DNA molecules 21 in the solution 111 bind to the avidin molecules bound to each DNA-attachable spot 101 on the support 100 (immobilization of biotinylated DNA). Since the numbers of the avidin molecules 22 immobilized to individual DNA-attachable spots 101 are equal, the numbers of probe DNA molecules which bind to the individual DNA-attachable spots 101 are the same (refer to Fig. 4).

The number of avidin molecules which bind to each DNA-attachable spot 101 can be changed as desired by changing the shape and size of the DNA-attachable spots 101 by altering the patterns of the photomask used in the exposing step as described above.

Accordingly, the number of probe DNA molecules 21 immobilized to each DNA-attachable spot 101 can also be changed as desired.

Furthermore, if there is a variation in the concentration of probe DNA of the solutions 111 spotted on each DNA-attachable spot 101, invariably the same number of probe DNA molecules 21 can be immobilized to the DNA-attachable spot 101. This significantly improves the reproducibility of the biomolecule microarray of the present invention being a spotted-type biomolecule microarrays.

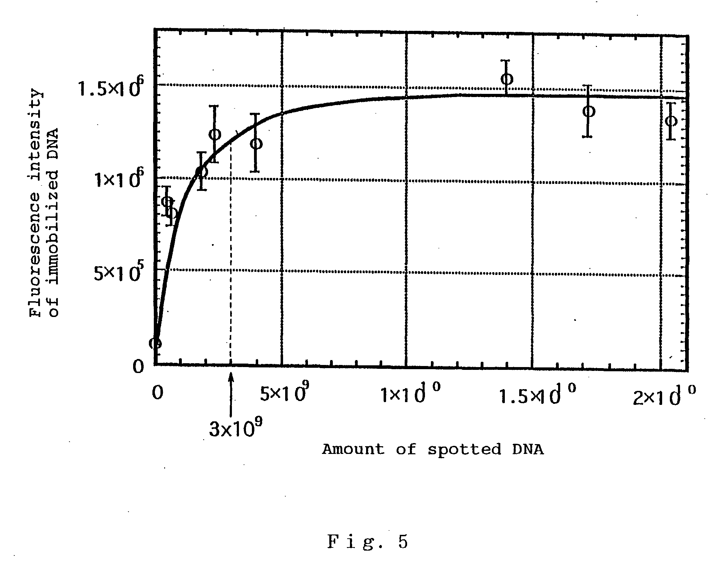

Fig. 5 shows a measured relationship between the amount of DNA (concentration) in the solution spotted on a DNA-attachable spot and the fluorescence intensity of DNA immobilized to the spot. It is known from the result of the measurement that if more than 3×10⁹ probe DNA molecules are spotted on a DNA-attachable spot, the amount of probe DNA immobilized to the spot becomes constant.

Since the DNA-attachable spots have a uniform size and shape and hence the same number of probe DNA molecules are immobilized to all the DNA-attachable spots, the biomolecule microarray of the present invention can be used for quantitative analysis.

Further, probe DNA binds only to the DNA-attachable spots, and attachment of probe DNA to the surface of the support other than the DNA-attachable spots by nonspecific adsorption can be prevented. As the result, noise (disturbing light) from around the DNA-attachable spots (detection spots) decreases, and the S/N ratio of fluorescence detection improves accordingly.

The S/N ratio of fluorescence detection can be further improved by forming the DNA-attachable spots (detection spots) in the same shape as that of the pixel elements of a semiconductor imaging device (CCD sensor or CMOS sensor, for example) used for image acquisition.

The present invention can also be realized in other embodiments.

For example, the surface-modified support of the present invention can be fabricated by another method different from the method shown in Fig. 1. In the fabricating method of Fig. 1, first an aluminum film 12 is formed on the surface of a slide glass 11, the aluminum film 12 on specific areas (spot-forming areas) 101 is removed by photolithography and etching to expose the glass surface, a DNA-immobilizing layer 15 is then formed on the surface of the support, and the remaining aluminum film 12 is dissolved with an etching solution and as a result the DNA-immobilizing layer remains only on the specific areas 101. In another method, first a DNA-immobilizing layer 15 is formed on the surface of a slide glass 11, and the DNA-immobilizing layer 15 is covered with an aluminum film 12 and exposed only on the specific areas 101. Specifically, first a DNA-immobilizing layer 15 and then an aluminum film 12 are formed on the entire surface of a slide glass 11 one over the other. Next, the aluminum film 12 is coated with a positive photoresist 13. The photoresist 13 only on the specific areas 101 is exposed to light by exposure through a photomask and removed by development. Finally, the aluminum film 12 on the specific areas 101 is removed by etching to expose the DNA-immobilizing layer 15 only on the specific areas 101.

Although a positive photoresist is used in the above-described embodiments, a negative type photoresist can also be used in combination of a negative photomask.

Further, not only a DNA microarray but also microarrays using RNA, PNA, or protein for probe biomolecules are included in the biomolecule microarray of the present invention, though a DNA microarray is described as an embodiment.

Further, the support used for the surface-modified support is not limited to a slide glass, and silica glass, silicon plate, plastic plate, gold or gold-coated plate, or silver or silver-coated plate.

Furthermore, instead of the biomolecule-attachable layer with avidin bound to the surface used in the above-described embodiment, any other layer best suited for immobilizing a fixed number of probe biomolecules can be used, taking into the binding between the layer and the probe biomolecules to immobilize.

As understood from the above description, the present invention has the following advantages:

The biomolecule microarray support of the present invention has a surface modified so that probe biomolecules attach only to predetermined specific areas (probe biomolecule-attachable spots) of the surface.

Because of this surface modification, attachment of the probe biomolecules in spotted solutions to the surface of the support outside the specific areas can be prevented.

Further, the number of the probe biomolecules immobilized to each specific area is determined by the size and shape of the specific area, and can be changed as desired by changing the size and shape of the specific area. The numbers of the probe biomolecules immobilized to all specific areas are therefore made equal by forming the specific areas in a uniform size and shape as the support is usually so made.

The biomolecule microarray of the present invention, made by spotting solutions containing probe biomolecules on the support of the present invention, can be used for quantitative analysis of target biomolecules because of its uniform and unvarying number of probe biomolecules immobilized to the detection spots. Further, since this biomolecule microarray has a biomolecule-attachable coating only on the specific areas (detection spots), nonspecific attachment of target biomolecules to the surface of the support other than the detection spots can be prevented, and hence the S/N ratio of measurement can be significantly improved.

Furthermore, the biomolecule microarray support of the present invention can be made precisely and efficiently using the photolithography technique by the support-fabricating method of the present invention.

The above preferred embodiments of the present invention are disclosed by way of example. Those skilled in the art will realize that various modifications and changes may be made within the sprit and scope of this invention. Hence the invention is not to be limited to the embodiments as described, but the invention encompasses the full range of equivalencies as defined by the appended claims.

## Claims

1. A biomolecule microarray support for spotting solutions containing probe biomolecules on the surface and immobilizing the probe biomolecules in the solutions to the surface, **characterized in that**
a plurality of small-sized probe biomolecule-attachable spots are arrayed in a regular arrangement on the surface of the support.

2. The biomolecule microarray support of claim 1, wherein said probe biomolecule-attachable spots have a layer of any one of biomolecule-immobilizing agents including avidin, streptavidin, biotin, amino group, carbonyl group, hydroxyl group, succinimide group, maleimide group, and thiol group.

3. The biomolecule microarray support of claim 1 or 2, wherein said support is a glass plate, silicon plate, plastic plate, gold or gold-coated plate, or silver or silver-coated plate.

4. The biomolecule microarray support of any one of claims 1 to 3, wherein said probe biomolecule-attachable spots have avidin molecules bound in a single layer to the ends of the biotin molecules bound to the surface of the support.

5. A biomolecule microarray **characterized in that** probe biomolecules are bound to said probe biomolecule-attachable spots of the support of any one of claims 1 to 4.

6. The biomolecule microarray of claim 5, wherein said probe biomolecules are DNA, RNA, PNA, or protein.

7. The biomolecule microarray of claim 5 or 6, wherein said probe biomolecules are biotin-labeled biomolecules and are bound to said probe biomolecule-attachable spots by biotin-avidin binding.

8. A method of fabricating the biomolecule microarray support of any one of claims 1 to 4, comprising steps by which said probe biomolecule-attachable spots are formed only on the specific areas of the surface of a support by the photolithography and etching technique.
